# EUROPEAN PATENT SPECIFICATION

(11) **EP 3 578 217 B1**
(45) Date of publication and mention of the grant of the patent: **23.12.2020**
(21) Application number: 18176848.2
(22) Date of filing: 08.06.2018
(51) Int. Cl.: A61M 15/00, B65D 83/30

(54) **INHALER**
INHALATOR
INHALATEUR

(43) Date of publication of application: 11.12.2019
(73) Proprietor: Presspart Manufacturing S.A., 43720 L'Arboc del Penedes, Tarragona (ES)
(72) Inventor: TORRES MUNIESA, Victor, 43720 L'Arboç, Tarragona (ES); HERRERA MARTÍNEZ, Paloma, 43720 L'Arboç, Tarragona (ES); BUSTO SOTIL, Santiago, 43720 L'Arboç, Tarragona (ES); VALENCIA PELLISA, David, 43720 L'Arboç, Tarragona (ES)
(74) Representative: Uexküll & Stolberg

(56) References cited:
- US-A1- 2001 013 342
- US-A1- 2013 133 643
- US-A1- 2015 122 257

## Description

### TECHNICAL FIELD

The present invention relates to an inhaler having an inhaler body for retaining an aerosol container with a dispensing valve at a valve end of the container. The inhaler body comprises a first open end sized and arranged to receive the aerosol container with a dispensing valve and a second open end through which a dose of medicament is dispensed as an aerosol. The second open end is sized and arranged to be coupled to the mouth or nasal cavities of a patient. The inhaler body further comprises a nozzle block which extends from a bottom part of the inhaler body and against which the dispensing valve of the aerosol container is depressed to release a dose of a medicament. The nozzle block has a fluid flow path extending therethrough such that the dose of medicament being released from the valve is passed to the nozzle block and exits the nozzle block as an aerosol in a direction towards the second open end of the inhaler body.

### BACKGROUND OF THE INVENTION

Inhalers, such as metered dose inhalers (MDIs), are medication delivery devices which deliver a pharmaceutical formulation including one or more pharmaceutical active compounds to a human or another mammalian patient. Typically, the pharmaceutical formulation is delivered by the MDIs in unit doses in the form of an aerosol. Each actuation of the MDIs delivers one unit dose. The unit dose is expelled by the MDIs and is taken into the body of the patient on inhalation via the nose or mouth. The pharmaceutical formulation is delivered to or via the respiratory tract, notably to the lungs, of the patient on inhalation. Metered dose inhalers are typically used for the treatment of respiratory infections and disorders, including respiratory tract infections, obstructive lung disease, inflammatory lung disease and chronic obstructive pulmonary disease. Asthma treatment is a particularly common use of MDIs.

ES 1069857 U discloses an inhaler with a notably cylindrical vertical part configured to receive a tank containing a medical product. A lower base of the vertical part protrudes perpendicularly forward to form an outlet opening for the medical product. The lower base of the vertical part encloses an angle of greater than 90° with the vertical part. An outlet structure is positioned on the lower base of the vertical part and comprises several holes in order to direct aerosol from the tank being received in the vertical part to the outlet opening.

Inhalers are preferably manufactured by injection molding where melted plastic is injected into moulds where it solidifies and takes the appearance of an inhaler body. In order to ensure proper functioning of the inhalers it has to be ensured that in particular dimensions of the nozzle block lie within a given specification. This is because the design of the nozzle block significantly influences the spray pattern of the aerosol exiting the nozzle towards the second open end of the inhaler body.

Typically, the dimensions of the nozzle block are measured by systems via contact measurement. However, due to their complex geometry some designs have to be measured indirectly. Alternatively, the inhaler body is cut in half in order to allow a direct measurement of the nozzle block and its interior design (destructive test).

Indirect measurements of nozzle block designs are often imprecise and/or are not feasible as an in-line inspection in the production process at reasonable complexity and costs. Moreover, destructive tests only allow the measurement of a limited number of parts out of a batch as naturally not all fabricated parts shall be destroyed for measurement purposes. Additionally, the precision of the cutting operation as well as post cutting deformations due to stress in the plastic parts may affect the measurement results.

US 2001/013342 A1 relates to an actuator for a pressurized metered dose inhaler and a pressurized metered dose inhaler including the same.

US 2013/133643 A1 relates to an actuator for an inhaler for administering medicament by inhalation and to an inhaler including the same.

US 2015/122257 A1 relates to an apparatus for nebulising medicinal formulations, wherein the nebulisation of the formulation is assisted by propellants.

It is an object of the present invention to provide an inhaler which allows direct measurement of the dimensions of the nozzle block without having to destroy the inhaler.

### SUMMARY OF THE INVENTION

This object is achieved by an inhaler comprising the features of claim 1, preferred embodiments are set out in the dependent claims.

According to the present invention, the nozzle block is formed of transparent material. A transparent nozzle block can be measured using vision systems which do not require any specific handling or physical change (e.g. cutting, adding liquids for increasing contrast, etc.). Vision systems typically allow to capture a digital image of a part which is to be measured.

The measurements are then taken from the image manually or automatically in a subsequent step. Preferably the vision system comprises a camera or any other device which is capable of taking an image of the part to be measured.

Preferably, the nozzle block is formed of transparent material whereas the remaining portion of the inhaler body is formed of nontransparent material. In this case the camera of the vision system may be at least partially inserted into the inhaler body in order to capture an image of the nozzle block. The measurement may then be taken from the image outside of the inhaler body.

Optionally, the whole inhaler body including the nozzle block is formed of transparent material. This allows measurement of the nozzle block dimensions through the inhaler body and thus from the outside of the inhaler body. Preferably, the inhaler body passes the camera of the vision system close by such that an image of the nozzle block is captured through an outer wall of the inhaler body. The measurements from the image may then be taken by another device such as a computing device.

In an embodiment according to the present invention, the nozzle block has a cylindrical shape and extends from the bottom part towards the first open end of the inhaler body. A cylindrical nozzle block is easy to manufacture and thus allows cheap tooling.

Preferably, the fluid flow path of the nozzle block is formed by a sump configured to receive the dispensing valve of the aerosol container, a nozzle configured to aerosolize the dose of medicament being released from the dispensing valve and a channel extending from the sump to the nozzle. In order to ensure that the molded inhalers are dimensionally within a given specification, it is amongst other dimensions of interest to measure the diameter of the channel in transition to the nozzle (orifice diameter) as well as the length of the channel (jet length) of the nozzle block. This ensures proper functioning of the nozzle block during inhalation.
In another embodiment the nozzle block has a cylindrical upper portion, a cylindrical lower portion and a middle portion positioned in between the upper portion and the lower portion wherein the middle portion comprises opposing recesses which each have a plane base surface. The base surfaces are arranged parallel to one another. The plane base surfaces allow radiation, in particular light radiation, to enter and exit the nozzle block at a right angle with regard to the plane base surfaces and thus without being deflected. This allows a non distorted image of the nozzle block to be captured and thus enables true measurements of the interior of the nozzle block. In an embodiment of the present invention the base surfaces of the recesses are arranged parallel to a first axis defined by the channel connecting the sump and the nozzle of the nozzle block. Additionally, the base surfaces are arranged parallel to a second axis defined by the nozzle block extending from the bottom part towards the first open end of the inhaler body. This allows direct measurement of the jet length as well as the orifice diameter of the channel without distortion. This enables a cost-effective, fast, 100% in-line inspection check for parts supplied to the customer, such that batch traceability is given.

According to the present invention, the nozzle block is formed as a part separate from the inhaler body and is insertable through an opening in the bottom part of the inhaler body with a nozzle block cover. The nozzle block cover is configured to close the opening of the bottom part upon insertion of the nozzle into the inhaler body. Forming the nozzle block as a part separate from the inhaler body is a cost effective way to individually design the nozzle block according to customer needs, whereas the design of the inhaler body remains the same. Customers often design the nozzle block differently due to the significant influence of the nozzle block design on the spray pattern of the aerosol exiting the nozzle towards the second open end of the inhaler body. Moreover, the nozzle block design depends amongst others on the formulation of the medicament to be used in combination with the inhaler. Forming the nozzle block as a part separate from the inhaler body additionally allows the nozzle block to be designed as a part being compatible with a range of inhaler bodies having different sizes and/or different shapes. Moreover, the design of the nozzle block can be optimized with regard to the manufacturing process, in particular with regard to the injection molding of the nozzle block, without having to consider producibility of the nozzle block and the inhaler body in one single mould. Additionally, separating the manufacturing of the inhaler body and the nozzle block allows cheaper tooling as well as a shorter cycle time for both the nozzle block and the inhaler body. Consequently, the production costs for an inhaler and thus the price of sale decreases.

In an embodiment of the present invention the nozzle block cover has a bottom side which is flush with a bottom side of the inhaler body. This arrangement not only enables an appealing appearance of the inhaler but also avoids disturbing edges in transition between the bottom side of the inhaler body and the bottom side of the nozzle block.

In another embodiment the cover comprises corrugations at its bottom side. The area of the nozzle block cover is often used by patients to hold the inhaler during inhalation. As the corrugations allow secure gripping of the inhaler during inhalation an accidently dropping of the inhaler is avoided.

According to another embodiment the cover is connected to the bottom part of the inhaler body via a form fitting connection. The form fitting connection is configured to withstand the actuation force when the canister is moved towards the bottom part of the inhaler body such that a dose of medicament is dispensed. The form fitting connection is thus configured to enable a secure connection of the nozzle block to the bottom part of the inhaler body. Moreover, the form fitting connecting preferably secures proper positioning of the nozzle block with regard to the second open end of the inhaler body. In particular the form fitting connection secures positioning of the nozzle block in the inhaler body such that the dose of medicament being released from the valve exits the nozzle block as an aerosol in a direction towards the second open end of the inhaler body. Additionally, the form fitting connection preferably allows an airtight connection between the nozzle block cover and the bottom part such that during inhalation no air uncontrollably exits or enters the inhaler body.

In another embodiment of the present invention, the form fitting connection is a snap fit connection. Snap fit connections allow easy and fast assembling of the nozzle block and the inhaler body. Preferably, the snap fit connection is realized by snap-in tongues positioned on the nozzle block cover and being configured to engage with corresponding counterparts of the inhaler body. Alternatively, the snap-in tongues are positioned on the inhaler body and are configured to engage with corresponding counterparts of the nozzle block cover.

The invention will now be described in connection with several examples shown in the figures in which:
Figure 1 shows a perspective view of an example of an inhaler according to the present disclosure,
Figure 2 shows a cross sectional view of the inhaler of Figure 1,
Figure 3 shows a perspective view of a nozzle block,
Figure 4 shows a front view of the nozzle block of Figure 3 and
Figure 5 shows a cross sectional view of an embodiment of an inhaler according to the present invention.

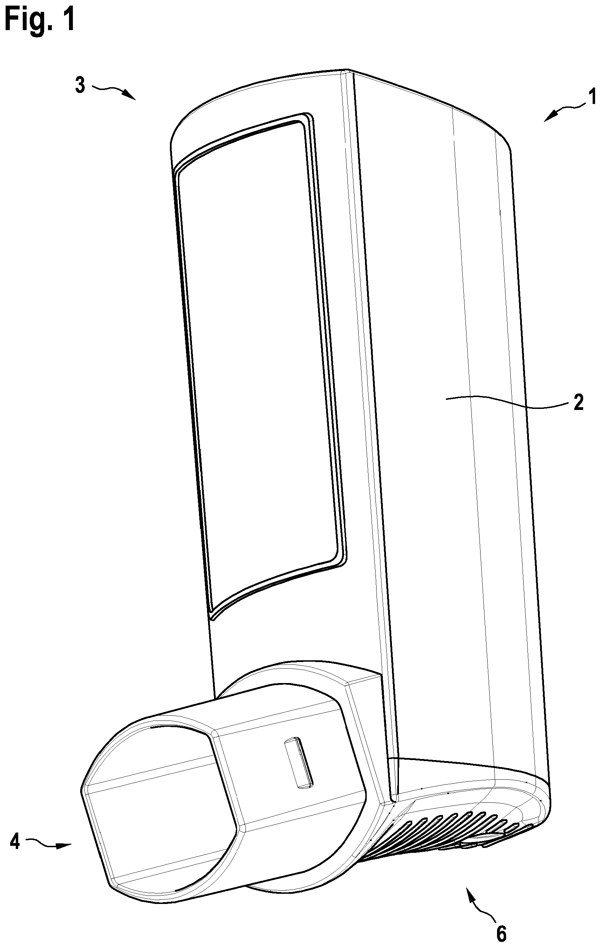

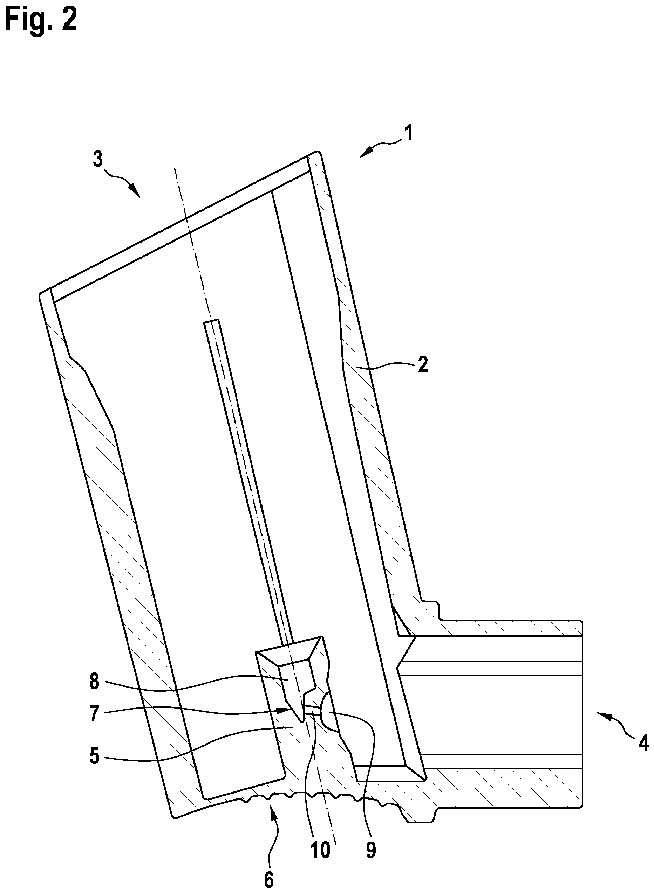

Figures 1 and 2 show an inhaler 1 having an inhaler body 2 for retaining an aerosol container (not shown) with a dispensing valve at a valve end of the container. The inhaler body 2 comprises a first open end 3 sized and arranged to receive the aerosol container with a dispensing valve and a second open end 4 through which a dose of a medicament is dispensed as an aerosol. The second open end 4 is sized and arranged to be coupled to the mouth or nasal cavities of a patient (not shown).

The inhaler body 2 further comprises a nozzle block 5 which extends from a bottom part 6 of the inhaler body 2 and against which the dispensing valve of the aerosol container is depressed to release a dose of medicament. The nozzle block 5 has a fluid flow path 7 extending therethrough such that the dose of medicament being released from the valve is passed to the nozzle block 5 and exits the nozzle block 5 as an aerosol in a direction towards the second open end 4 of the inhaler body 2. The fluid flow path 7 of the nozzle block 5 is formed by a sump 8 configured to receive the dispensing valve of the aerosol container, a nozzle 9 configured to aerosolize the medicament being released from the dispensing valve and a channel 10 extending from the sump 8 to the nozzle 9.

The nozzle block 5 is formed of transparent material, whereas the remaining portion of the inhaler body is formed of nontransparent material.

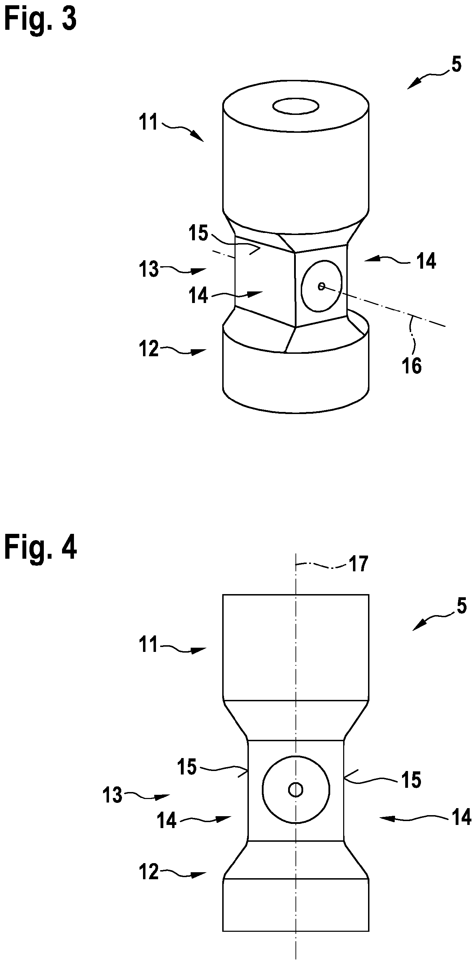

Figures 3 and 4 show the nozzle block 5 in more detail. The nozzle block 5 has a cylindrical upper portion 11, a cylindrical lower portion 12 and a middle portion 13 positioned in between the upper portion 11 and the lower portion 12. The middle portion 13 comprises opposing recesses 14 which each have a plane base surface 15. The base surfaces 15 are arranged parallel to a first axis 16 defined by the channel 10 connecting the sump 8 and the nozzle 9 of the nozzle block 5. Moreover, the base surfaces 15 are arranged parallel to a second axis 17 defined by the nozzle block 5 extending from the bottom part 6 towards the first open end of the inhaler body 2 (Fig. 2) .

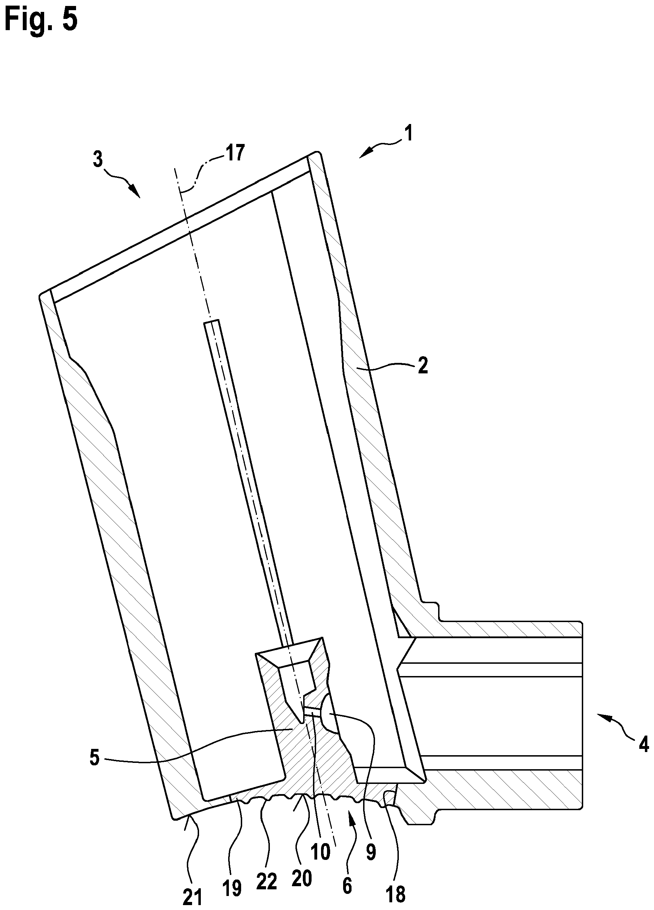

Figure 5 shows an embodiment of the inhaler 1. The inhaler 1' differs from the one shown in Figures 1 and 2 in that the nozzle block 5 is formed as a part separate from the inhaler body 2 and is insertable through an opening 18 in the bottom part 6 of the inhaler body 2 together with a nozzle block cover 19. The nozzle block cover 19 is configured to close the opening 18 of the bottom part 6 upon insertion of the nozzle block 5 into the inhaler body 2.

The nozzle block cover 19 has a bottom side 20 which is flush with a bottom side 21 of the inhaler body 2. The cover 19 comprises corrugations 22 at its bottom side 20.

The cover 19 is connected to the bottom part 6 of the inhaler body 2 via a snap fit connection. Preferably, the cover 19 comprises snap-in tongues which are designed to engage with corresponding counterparts of the inhaler body 2.

### REFERENCE NUMEREALS

- 1: Inhaler
- 2: Inhaler body
- 3: First open end
- 4: Second open end
- 5: Nozzle block
- 6: Bottom part
- 7: Fluid flow path
- 8: Sump
- 9: Nozzle
- 10: Channel
- 11: Upper portion
- 12: Lower portion
- 13: Middle portion
- 14: Recesses
- 15: Base surface
- 16: First axis
- 17: Second axis
- 18: Opening
- 19: Nozzle block cover
- 20: Bottom side (cover)
- 21: Bottom side (inhaler body)
- 22: Corrugations (cover)

## Claims

1. An inhaler having an inhaler body (2) for retaining an aerosol container with a dispensing valve at a valve end of the container, the inhaler body (2) comprising:
- a first open end (3) sized and arranged to receive the aerosol container with a dispensing valve;
- a second open end (4) through which a dose of medicament is dispensed as an aerosol, the second open end (4) being sized and arranged to be coupled to the mouth or nasal cavities of a patient;
- a nozzle block (5) which extends from a bottom part (6) of the inhaler body (2) and against which the dispensing valve of the aerosol container is depressed to release a dose of a medicament, the nozzle block (5) having a fluid flow path (7) extending therethrough such that the dose of medicament being released from the valve is passed to the nozzle block (5) and exits the nozzle block (5) as an aerosol in a direction towards the second open end (4) of the inhaler body (2);
**characterized in**
**that** the nozzle block (5) is formed of transparent material and
**that** the nozzle block (5) is formed as a part separate from the inhaler body (2) and is insertable through an opening (18) in the bottom part (6) of the inhaler body (2) together with a nozzle block cover (19) which is configured to close the opening (18) of the bottom part (6) upon insertion of the nozzle block (5) into the inhaler body (2).

2. Inhaler according to claim 1, **characterized in that** the nozzle block (5) has a cylindrical shape and extends from the bottom part (6) towards the first open end (3) of the inhaler body (2).

3. Inhaler according to any one of the preceding claims, **characterized in that** the fluid flow path (7) of the nozzle block is formed by a sump (8) configured to receive the dispensing valve of the aerosol container, a nozzle (9) configured to aerosolize the dose of medicament being released from the dispensing valve and a channel (10) extending from the sump (8) to the nozzle (9).

4. Inhaler according to any one of the claims 1 or 3, **characterized in that** the nozzle block (5) has a cylindrical upper portion (11), a cylindrical lower portion (12) and a middle portion (13) positioned in between the upper portion (11) and the lower portion (12), wherein the middle portion (13) comprises opposing recesses (14) which each have a plane base surface (15), wherein the base surfaces (15) are arranged parallel to one another.

5. Inhaler according to claim 4 insofar as dependent on claim 3, **characterized in that** the base surfaces (15) of the recesses (14) are arranged parallel to a fist axis (16) defined by the channel (10) connecting the sump (8) and the nozzle (9) of the nozzle block (5) and are arranged parallel to a second axis (17) defined by the nozzle block (5) extending from the bottom part (6) towards the first open end (3) of the inhaler body (2) .

6. Inhaler according to claim 1, wherein the nozzle block cover (19) has a bottom side (20) which is flush with a bottom side (21) of the inhaler body (2).

7. Inhaler according to claim 1, wherein the cover (19) comprises corrugations (22) at its bottom side (20).

8. Inhaler according to claim 1, **characterized in that** the cover (19) is configured to be connected to the bottom part (6) of the inhaler body (2) via a form fitting connection.

9. Inhaler according to claim 8, **characterized in that** the form fitting connection is a snap fit connection.

10. Inhaler according to any of the preceding claims, **characterized in that** the inhaler comprises a detachment mechanism configured to allow detachment of the nozzle block (5) from the inhaler body (2) upon manual actuation, wherein the nozzle block (5) is configured to be reusable in combination with another inhaler.

## Patentansprüche

1. Inhalator mit einem Inhalatorgehäuse (2) zum Halten eines Aerosol-Behälters mit einem Dosierventil an einem Ventilende des Behälters, wobei das Inhalatorgehäuse (2) umfasst:
- ein erstes offenes Ende (3), das dimensioniert und angeordnet ist, um den Aerosol-Behälter mit einem Dosierventil aufzunehmen;
- ein zweites offenes Ende (4), durch das eine Medikamentendosis als ein Aerosol abgegeben wird, wobei das zweite offene Ende (4) dimensioniert und angeordnet ist, um mit dem Mund oder Nasenhöhlen eines Patienten gekoppelt zu werden;
- ein Düsenblock (5), der sich von einem Bodenteil (6) des Inhalatorgehäuses (2) erstreckt und gegen den das Dosierventil des Aerosol-Behälters gedrückt wird, um eine Medikamentendosis freizugeben, wobei der Düsenblock (5) einen Fluidflussweg (7) aufweist, der sich durch diesen erstreckt, so dass die Medikamentendosis, die von dem Ventil freigegeben wird, zum Düsenblock (5) weitergegeben wird und den Düsenblock (5) als ein Aerosol in Richtung des zweiten offenen Endes (4) des Inhalatorgehäuses (2) verlässt;
**dadurch gekennzeichnet,**
**dass** der Düsenblock (5) aus transparentem Material geformt ist und
**dass** der Düsenblock (5) als ein von dem Inhalatorgehäuse (2) separates Teil ausgebildet ist, und durch eine Öffnung (18) in dem Bodenteil (6) des Inhalatorgehäuses (2) zusammen mit einer Düsenblockabdeckung (19) einsetzbar ist, die ausgebildet ist, die Öffnung (18) des Bodenteils (6) beim Einsetzen des Düsenblocks (5) in das Inhalatorgehäuse (2) zu schließen.

2. Inhalator nach Anspruch 1, **dadurch gekennzeichnet, dass** der Düsenblock (5) eine zylindrische Form hat und sich von dem Bodenteil (6) in Richtung des ersten offenen Endes (3) des Inhalatorgehäuses (2) erstreckt.

3. Inhalator nach einem der vorhergehenden Ansprüche, **dadurch gekennzeichnet, dass** der Fluidflussweg (7) des Düsenblocks durch einen Sammelbehälter (8), der eingerichtet ist, das Dosierventil des Aerosol-Behälters aufzunehmen, eine Düse (9), die eingerichtet ist, die Medikamentendosis, die von dem Dosierventil freigegeben wird, zu aerosolisieren, und einen Kanal (10) gebildet ist, der sich von dem Sammelbehälter zu der Düse (9) erstreckt.

4. Inhalator nach einem der Ansprüche 1 oder 3, **dadurch gekennzeichnet, dass** der Düsenblock (5) einen zylindrischen oberen Abschnitt (11), einen zylindrischen unteren Abschnitt (12) und einen mittleren Abschnitt (13) hat, der zwischen dem oberen Abschnitt (11) und dem unteren Abschnitt (12) positioniert ist, wobei der mittlere Abschnitt (13) gegenüberliegende Aussparungen (14) aufweist, die jeweils eine plane Grundfläche (15) aufweisen, wobei die Grundflächen (15) parallel zueinander angeordnet sind.

5. Inhalator nach Anspruch 4, insoweit abhängig von Anspruch 3, **dadurch gekennzeichnet, dass** die Grundflächen (15) der Aussparungen (14) parallel zu einer ersten Achse (16) angeordnet sind, die durch den Kanal (10), der den Sammelbehälter (8) und die Düse (9) des Düsenblocks (5) verbindet, definiert ist, und parallel zu einer zweiten Achse (17) angeordnet sind, die durch den Düsenblock (5) definiert ist, der sich von dem Bodenteil (6) in Richtung des ersten offenen Endes (3) des Inhalatorgehäuses (2) erstreckt.

6. Inhalator nach Anspruch 1, wobei die Düsenblockabdeckung (19) eine Bodenseite (20) aufweist, die bündig mit einer Bodenseiten (21) des Inhalatorgehäuses (2) ist.

7. Inhalator nach Anspruch 1, wobei die Abdeckung (19) Riffelungen (22) an ihrer Bodenseite (20) aufweist.

8. Inhalator nach Anspruch 1, **dadurch gekennzeichnet, dass** die Abdeckung (19) ausgebildet ist, mit dem Bodenteil (6) des Inhalatorgehäuses (2) mittels einer formschlüssigen Verbindung verbunden zu werden.

9. Inhalator nach Anspruch 8, **dadurch gekennzeichnet, dass** die formschlüssige Verbindung eine Schnappverbindung ist.

10. Inhalator nach einem der vorhergehenden Ansprüche, **dadurch gekennzeichnet, dass** der Inhalator einen Trennmechanismus aufweist, der ausgebildet ist, ein Trennen des Düsenblocks (5) von dem Inhalatorgehäuse (2) bei manueller Betätigung zu ermöglichen, wobei der Düsenblock (5) ausgebildet ist, in Kombination mit einem anderen Inhalator wieder verwendet zu werden.

## Revendications

1. Inhalateur présentant un corps d'inhalateur (2) pour retenir un contenant d'aérosol avec une valve distributrice à une extrémité de valve du contenant, le corps d'inhalateur (2) comprenant :
- une première extrémité ouverte (3) dimensionnée et agencée pour recevoir le contenant d'aérosol avec une valve distributrice ;
- une seconde extrémité ouverte (4) à travers laquelle une dose de médicament est distribuée en tant qu'un aérosol, la seconde extrémité ouverte (4) étant dimensionnée et agencée pour être couplée aux cavités buccale ou nasale d'un patient ;
- un bloc de buse (5) qui s'étend d'une partie inférieure (6) du corps d'inhalateur (2) et contre lequel la valve distributrice du contenant d'aérosol est appuyée pour libérer une dose d'un médicament, le bloc de buse (5) présentant un trajet d'écoulement de fluide (7) s'étendant au travers de façon à ce que la dose de médicament en train être libérée de la valve soit dirigée vers le bloc de buse (5) et sorte du bloc de buse (5) en tant qu'un aérosol dans une direction vers la seconde extrémité ouverte (4) du corps d'inhalateur (2) ;
**caractérisé en ce que**
le bloc de buse (5) est formé de matériau transparent et
que le bloc de buse (5) est formé en tant qu'une partie séparée du corps d'inhalateur (2) et peut être inséré à travers une ouverture (18) dans la partie inférieure (6) du corps d'inhalateur (2) conjointement à un couvercle de bloc de buse (19) qui est configuré pour fermer l'ouverture (18) de la partie inférieure (6) à l'insertion du bloc de buse (5) dans le corps d'inhalateur (2).

2. Inhalateur selon la revendication 1, **caractérisé en ce que** le bloc de buse (5) a une forme cylindrique et s'étend de la partie inférieure (6) en direction de la première extrémité ouverte (3) du corps d'inhalateur (2) .

3. Inhalateur selon l'une quelconque des revendications précédentes, **caractérisé en ce que** le trajet d'écoulement de fluide (7) du bloc de buse est formé par une cuvette (8) configurée pour recevoir la valve distributrice du récipient d'aérosol, une buse (9) configurée pour transformer en aérosol la dose de médicament en train d'être libérée de la valve distributrice et un canal (10) s'étendant de la cuvette (8) vers la buse (9).

4. Inhalateur selon l'une quelconque des revendications 1 ou 3, **caractérisé en ce que** le bloc de buse (5) présente une partie supérieure cylindrique (11), une partie inférieure cylindrique (12) et une partie centrale (13) positionnée entre la partie supérieure (11) et la partie inférieure (12), dans lequel la partie centrale (13) comprend des cavités opposées (14) qui ont chacune une surface de base plane (15), dans lequel les surfaces de base (15) sont agencées parallèlement l'une à l'autre.

5. Inhalateur selon la revendication 4 dans la mesure où dépendante de la revendication 3, **caractérisé en ce que** les surfaces de base (15) des cavités (14) sont agencées parallèlement à un premier axe (16) défini par le canal (10) reliant la cuvette (8) et la buse (9) du bloc de buse (5) et sont agencées parallèlement à un second axe (17) définit par le bloc de buse (5) s'étendant de la partie inférieure (6) en direction de la première extrémité ouverte (3) du corps d'inhalateur (2).

6. Inhalateur selon la revendication 1, dans lequel le couvercle de bloc de buse (19) présente une face inférieure (20) qui affleure avec une face inférieure (21) du corps d'inhalateur (2).

7. Inhalateur selon la revendication 1, dans lequel le couvercle (19) comprend des ondulations (22) sur sa face inférieure (20).

8. Inhalateur selon la revendication 1, **caractérisé en ce que** le couvercle (19) est configuré pour être relié à la partie inférieure (6) du corps d'inhalateur (2) par le biais d'une connexion par ajustement de formes.

9. Inhalateur selon la revendication 8, **caractérisé en ce que** la connexion par ajustement de formes est une connexion à pression.

10. Inhalateur selon l'une quelconque des revendications précédentes, **caractérisé en ce que** l'inhalateur comprend un mécanisme de séparation configuré pour permettre la séparation du bloc de buse (5) du corps d'inhalateur (2) par actionnement manuel, dans lequel le bloc de buse (5) est configuré pour être réutilisable en combinaison avec un autre inhalateur.
